# EUROPEAN PATENT SPECIFICATION

(11) **EP 3 113 840 B1**
(45) Date of publication and mention of the grant of the patent: **19.12.2018**
(21) Application number: 15710372.2
(22) Date of filing: 04.03.2015
(51) Int. Cl.: A61N 1/375, C23C 14/00

(54) **IMPLANTABLE MEDICAL DEVICE HAVING A CONDUCTIVE COATING**
IMPLANTIERBARE MEDIZINISCHE VORRICHTUNG MIT LEITFÄHIGER BESCHICHTUNG
DISPOSITIF MÉDICAL IMPLANTABLE AYANT UN REVÊTEMENT CONDUCTEUR

(30) Priority: 07.03.2014 US 201461949617 P
(43) Date of publication of application: 11.01.2017
(73) Proprietor: Cameron Health, Inc., St. Paul, Minnesota 55112 (US)
(72) Inventor: KANE, Michael J., Roseville, Minnesota 55113 (US); JONES, Matthew P., Shoreview, Minnesota 55126 (US); GROPEN, Sverre, Minneapolis, Minnesota 55407 (US)
(74) Representative: Pfenning, Meinig & Partner mbB
(86) International application number: PCT/US2015/018779
(87) International publication number: WO 2015/134636

(56) References cited:
- US-A1- 2003 083 697
- US-A1- 2005 288 733
- US-A1- 2009 047 413
- US-A1- 2010 310 756

## Description

### TECHNICAL FIELD

This document relates generally to medical devices and, in particular, to a medical device having a conductive coating.

### BACKGROUND

Implantable medical devices (IMDs) can perform a variety of diagnostic or therapeutic functions. In an example, an IMD can include one or more cardiac function management features, such as to monitor the heart or to provide electrical stimulation (e.g., therapy) to a heart or to the nervous system. The cardiac function management features can be used to diagnose or treat a subject, for example, in cases of electrical or mechanical abnormalities of the heart. Examples of IMDs can include pacers, automatic implantable cardioverter-defibrillators (ICDs), cardiac resynchronization therapy (CRT) devices, implantable monitors, neuromodulation devices (e.g., deep brain stimulators, or other neural stimulators), cochlear implants, or drug pumps, among other examples.

Such IMDs can include electronic circuitry that can be carried by a housing or "can" that can be made of a biocompatible material, such as titanium. The housing carrying the electronic circuitry can be configured to wirelessly transfer information between implanted IMDs, or between an IMD and an assembly external to the body. Such information can include, for example, programming instructions or configuration information to configure the IMD to monitor, diagnose, or treat a physiologic condition. Such information can also include data sensed, detected, or processed by the IMD and transmitted to another device or assembly (e.g., physiologic information, a disease status, etc.). Document US 2005/0288733 A1 relates to an implantable electronic device formed within a biocompatible hermetic package. The implantable electronic device is used for a visual prosthesis for the restoration of sight in patients with lost or degraded visual function. The package may include a hard hermetic box, a thin film hermetic coating, or both.

### OVERVIEW

Generally, implantable medical devices (IMDs) can include a pacemaker, a defibrillator, a cardiac resynchronization therapy device, a neurostimulation device, an implantable monitoring device, or one or more other devices. An IMD may generate an electrostimulation (e.g, therapy) to be delivered to a desired tissue site. Delivery of the electrostimulation may be via electrodes that may be included as a portion of an implantable lead assembly.

The present inventors have recognized, among other things, a need to reduce the post-shock recovery time of IMDs, while maintaining or increasing the capacitance and color and abrasion characteristics. For example, after the electrostimulation (e.g., a charge) is delivered to the desired tissue site, the tissue can retain a portion of the charge and prevent the IMD from sensing the patient (e.g., heart rhythm). The IMD can begin sensing the patient after the charge retained in the patient has dissipated. The post-shock recovery time is a time after the delivered therapy that the IMD can sense the patient.

Previous approaches include a housing that does not include a coating on the housing. In some examples, the housings that do not include a coating can have a post-shock recovery time of about 10 seconds. Some other previous approaches can include a coating such as a single titanium nitride homogenous layer. The single homogenous titanium nitride layer can allow for a range of characteristics, for example, size, porosity, regularity, branching, isotropy, etc. However, a layer of titanium nitride, having coarse grain structure with large branching and high porosity characteristics can provide an high capacitance, but may have undesirable color and abrasion characteristics as compared to a layer of titanium nitride having fine grain sizes, lower porosity and limited branching structure (dominantly columnar structure). A titanium nitride layer having a fine grain structure with limited branching and dominantly columnar structure can provide increased color and abrasion characteristics but a decrease in capacitance as compared to the formerly described coarse grain structure with large branching and high porosity characteristics. Thus, the previous approaches including the single homogenous titanium nitride layer can be limited by either capacitance or color and abrasion characteristics depending on the structure.

Various embodiments of the present disclosure can provide an implantable medical device including a coating such as a conductive coating. The coating can include a layer of large grain titanium nitride and a layer of small grain titanium nitride. The combination of both large grain size and small grain size can provide the benefits of each grain size while minimizing or preventing the disadvantages associated with each grain size. The coating can decrease impedance by increasing a surface area of the coating, which can improve the post-shock recovery. The coating can increase the color and abrasion characteristics. Therefore, the coating of the present disclosure can decrease the post-shock recovery time of the implantable medical device while maintaining color and abrasion characteristics.

The implantable medical device of the present disclosure can include a metallic outer surface and a coating formed on at least a portion of the metallic outer surface. The coating can include an adhesion layer formed on at least a portion of the metallic outer surface, a titanium nitride base layer formed on at least a portion of the adhesion layer, a titanium intermediate layer formed on at least a portion of the titanium nitride base layer, and a titanium nitride top layer formed on at least a portion of the titanium intermediate layer. The IMDs and methods described herein have flexible manufacturing and a low cost of implementation.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, the various examples discussed in the present document.
FIG. 1 illustrates generally an example of a system including an IMD.
FIG. 2 illustrates generally a cross-section of an IMD including a coating.
FIG. 3 illustrates generally an example of a method of coating an implantable medical device.
FIG. 4 illustrates a cross-section scanning electron microscope (SEM) image of a portion of an IMD.
FIG. 5 illustrates a cross-section SEM image of a titanium nitrate base layer.
FIG. 6 illustrates a top view SEM image of the titanium nitrate base layer.
FIG. 7 illustrates a top view SEM image of an IMD including a coating.
FIG. 8 illustrates a graph representing the post-shock recovery of a plurality of IMDs.
FIG. 9 illustrates a graph representing the electrochemical impedance spectroscopy of Example 1 and Comparative Example 1.
FIG. 10 illustrates a graph representing the post-shock recovery of Example 1 and Comparative Example 1.
FIG. 11 illustrates a graph representing the post-shock recovery of Example 2 and Comparative Example 2.
FIG. 12 illustrates a graph representing the effect of a coating thickness on electrochemical impedance spectroscopy.

### DETAILED DESCRIPTION

The present disclosure describes, among other things, an implantable medical implant including a coating and related method. The implantable medical device can include a metallic outer surface and a coating formed on at least a portion of the metallic outer surface. The coating can include an adhesion layer formed on at least a portion of the metallic surface, a titanium nitride base layer formed on at least a portion of the adhesion layer, a titanium intermediate layer formed on at least a portion of the titanium nitride base layer, and a titanium nitride top layer formed on at least a portion of the titanium intermediate layer.

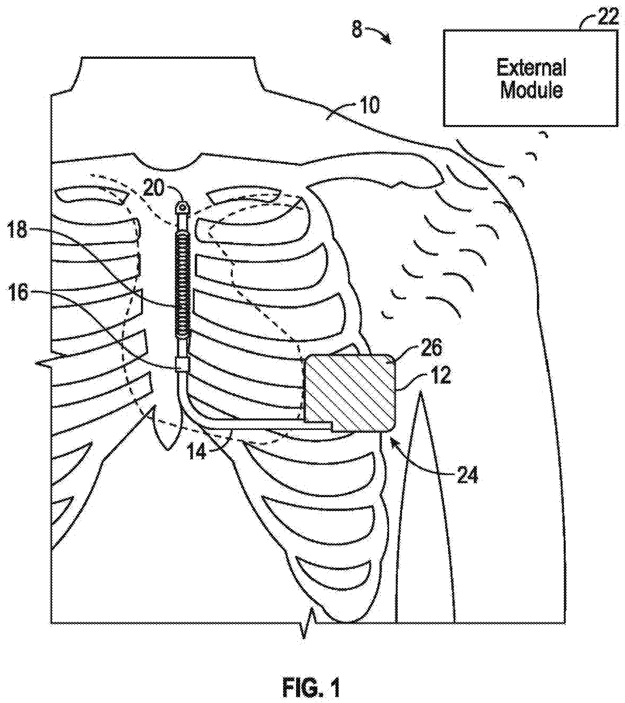

FIG. 1 illustrates generally an example of a system 8 that can include an implantable medical device (IMD) 24 implanted within a body (e.g., a patient 10), wirelessly coupled to an external module 22. The IMD 24 illustrated in FIG. 1 is a subcutaneous-only defibrillator. In an example, the IMD 24 can include an implantable device housing 12 (also referred to herein as "housing 12") and at least one lead 14 including one or more electrodes 16, 18, and 20. The housing 12 can be implanted in a patient 10, over the patient's ribs and beneath the skin. In an example, the implantable device housing 12 can be implanted, in the example, at approximately the left axilla (armpit), beneath the arm. The at least one lead 14 can extend from the housing 12 toward the patient's xiphoid and extends along and to the left of the sternum. The lead 14 includes electrodes 16, 18 and 20, with electrode 18 illustrated as a coil electrode designed primarily for shock delivery (though sensing via coil electrode 18 may be performed as well). The other electrodes 16 and 20 on lead 14 are shown as ring and cap electrodes, respectively. Other designs may be used.

The housing 12 can include a conductive surface or, if desired, has an area on its surface which is conductive to allow for at least sensing of electrical signals and, when needed, therapy delivery. The housing 12 can include a coating 26 (e.g., a conductive coating). As discussed herein, the coating can increase electrical properties (e.g., post-shock recovery), while maintaining the abrasion and color characteristics. In an example, the coating 26 can be used on the housing 12. In other examples, the coating can be applied to portions of the housing 12 and on the electrodes 16, 18 and/or 20. The housing 12 can be a hermetically-sealed titanium housing, or a housing including one or more other materials. The housing 12 can contain at least a portion of an implantable circuitry, such as a transmitter, a receiver, or a transceiver.

In an example, the housing 12 can be configured to mechanically and electrically couple the one or more leads 14 to the implantable circuitry of the housing 12. For example, the housing 12 can include an antenna configured to wirelessly transfer information electromagnetically to an external module 22. In an example, the external module 22 can include an external antenna coupled to an external telemetry circuit.

In an example, the external module 22 can include a physician programmer, a bedside monitor, or other relatively nearby assembly used to transfer programming instructions or configuration information to the IMD 24, or to receive diagnostic information, a disease status, information about one or more physiologic parameters, or the like, from the IMD 24. The external module 26 can be communicatively connected to one or more other external assemblies, such as a remote external assembly, located elsewhere (e.g., a server, a client terminal such as a web-connected personal computer, a cellular base-station, or another wirelessly-coupled or wired remote assembly). Other configurations of IMDs can be used and the coating can be applied to other components of the IMD. For example, the coating can be applied to electrode leads, ring electrodes, tip electrodes, and selected or discrete regions of implantable device housing, among others. Other system can also be used. For example, right-sided, anterior-posterior or other subcutaneous-only implantation, transvenous systems, epicardial systems, intravascular systems, leadless pacing devices, and hybrids/combinations thereof. Other implementations can include drug pumps or neurostimulation systems.

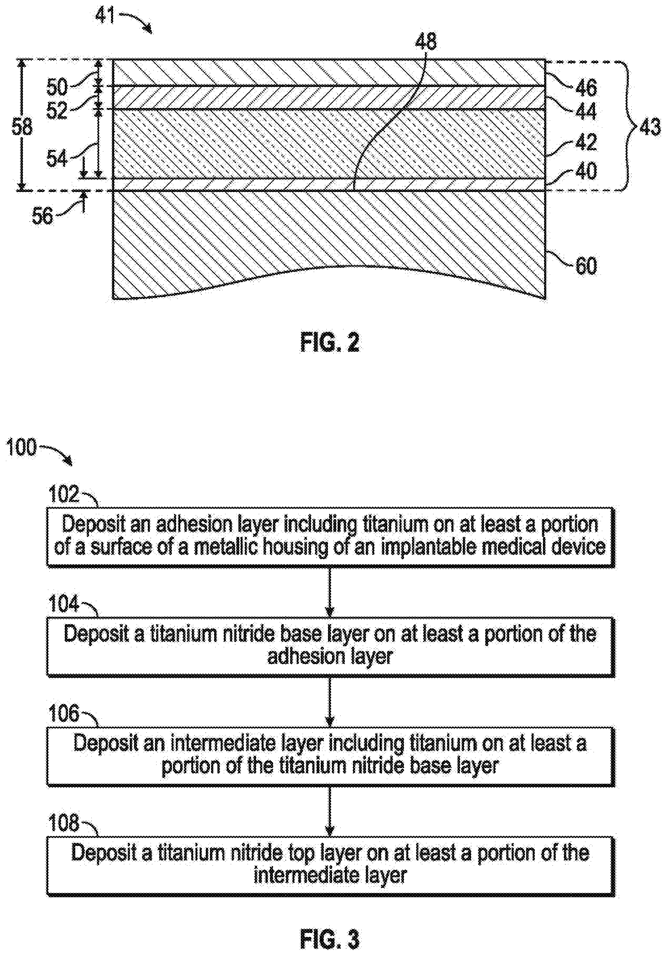

FIG. 2 illustrates generally a cross-section of an IMD 41 including a metallic housing 60 and a coating 43. In an example, the IMD 41 can include a pulse generator housing or "can," that can be used in a cardiac or other electrostimulation device to house one or more components and optionally to use or convey an electric pulse. In an example, the metallic housing 60 can be the housing 12 of the IMD 24, as shown in FIG 1. In an example, the structure represented by cross section drawing of IMD 41 can be employed in, including, but not limited to, at least one of a subcutaneous-only defibrillator, a pulse generator can, ring electrodes, tip electrodes, reference electrodes, cuff electrodes, fixated electrodes, cuff electrodes, patch electrodes, needle electrodes, mapping electrodes, catheter deployed intra-cardiac electrodes, intracranial stimulating and recording electrodes, neurostimulators, implantable recording systems (e.g., implantable loop recorders), and patient monitors, among others.

The IMD 41 can include a metallic housing 60 having a surface 48. In an example, the IMD 41 can be an electrode surface that can also be a pulse generator can. The metallic housing 60 can include a metallic material. The metallic material can include a biocompatible material, such as stainless steel, gold, silver, cobalt-chromium, platinum, iridium, palladium, titanium-based, or one or more combinations thereof. The IMD 41 can include the coating 43 formed on at least a portion of the metallic housing 60.

The coating 43 can include an adhesion layer 40, a base layer 42, an intermediate layer 44, and a top layer 46. The coating 43 can have a coating thickness within a range of about 0.0015 micrometers (µm) to about 50 µm. For example, the coating thickness can be within a range of about .25 µm to about 34 µm, such as for example 2.5 µm, 7.5 µm, and 15 µm. In an example, the coating 43 can include a base layer thickness 54 that is greater than an adhesion layer thickness 56, an intermediate layer thickness 52, and a top layer thickness 50. In an example, the top layer thickness 50 can be greater than an adhesion layer thickness 56 and the intermediate layer thickness 52.

The adhesion layer 40 can provide bonding between the metallic housing 60 and the base layer 42. The adhesion layer 40 can be formed on at least a portion of a surface 48 of the metallic housing 60 of the IMD 41. The adhesion layer 40 can include, but is not limited to, substantially pure titanium, ruthenium, vanadium, iridium, tantalum, niobium, tungsten, chromium, molybdenum, palladium, platinum, and combinations thereof. In an example, the titanium, ruthenium, vanadium, iridium, tantalum, niobium, tungsten, chromium, molybdenum, palladium, platinum, can be alloys and layered structures. In an example, the adhesion layer 40 includes substantially pure titanium. The term "substantially pure" as used herein refers to a majority of, or mostly, as in at least about 50%, 60%, 70%, 80%, 90%, 95%, 96%, 97%, 98%, 99%, 99.5%, 99.9%, 99.99%, or at least about 99.999% or more.

In an example, the adhesion layer 40 can have an adhesion layer thickness 56 within a range of about .10 % to about 75 % of the coating thickness 58. For example, the adhesion layer thickness 56 can be within a range of about 1 % to about 5 %, such as 1.5 %, 2.5 %, and 4 %, of the coating thickness 58. In an example, the adhesion layer thickness 56 can be within a range of about 0.0004 µm to about 38 µm. For example, the adhesion layer thickness 56 can be within a range of from .01 µm to about 5 µm, such as. 13 µm, .20 µm, and .26 µm. In an example, the adhesion layer thickness 56 can be within a range of 0.0004 µm to about 2 µm.

The base layer 42 can be formed on at least a portion of the adhesion layer 40. The base layer 42 can provide the core electrical properties and the film morphology of the coating 43. The base layer 42 can include, but is not limited to, porous metals, metal oxides, and metal nitrides. For example, the base layer 42 can include, but is not limited to, porous ruthenium, porous vanadium, porous iridium, porous tantalum, porous niobium, porous tungsten, porous molybdenum, porous palladium, porous platinum, porous titanium, ruthenium oxide, ruthenium nitride, titanium oxide, vanadium oxide, vanadium nitride, iridium nitride, iridium oxide, tantalum nitride, tantalum oxide, niobium nitride, niobium oxide, tungsten nitride, titanium nitride, tungsten oxide, titanium oxide, molybdenum oxide, molybdenum nitride, palladium oxide, palladium nitride, platinum oxide, platinum nitride, and combinations thereof. In an example, the base layer 42 can include titanium nitride and the base layer 42 can be referred to herein as the "titanium nitride base layer 42."

The base layer 42 can have a large grain size. The term "large grain size" as used herein refers to an average grain size that is greater than about .125 µm. In an example, the base layer 42 includes an average grain size within a range of about.125 µm to about 10 µm. For example, an average grain size of the base layer 42 can be within a range of about .126 µm to about 5µm, such as.5 µm, 1.5 µm, and 2.5 µm.

In an example, the base layer 42 can have a base layer thickness 54 within a range of about 1 % to about 99 % of the coating thickness 58. For example, the base layer thickness 54 can be within a range of about 60 % to about 95 %, such as 75 %, 83 %, and 90 % of the coating thickness 54. In an example, the base layer thickness 54 can be within a range of about .001 µm to about 49 µm. For example, the base layer thickness 54 can be within a range of from .002 µm to about 20 µm and 2 µm to about 25µm, such as 4.16 µm, 6.24 µm and 8.32 µm.

The intermediate layer 44 can be formed on at least a portion of the base layer 42. The intermediate layer 44 can provide bonding between the base layer 42 and the top layer 46 and improve abrasion resistance of the coating 43.

The intermediate layer 44 can include, but is not limited to, substantially pure titanium, ruthenium, vanadium, iridium, tantalum, niobium, tungsten, chromium, molybdenum, palladium, platinum, and combinations thereof. In an example, the titanium, ruthenium, vanadium, iridium, tantalum, niobium, tungsten, chromium, molybdenum, palladium, platinum, can be alloys and layered structures. In an example, the intermediate layer 44 includes substantially pure titanium and the intermediate layer 44 can be referred to herein as the "titanium intermediate layer 44."

In an example, the intermediate layer 44 can have an intermediate layer thickness 52 within a range of about .001 % to about 40 % of the coating thickness 58. For example, the intermediate layer thickness 52 can be within a range of about 0.5 % to about 25 %, such as 4 %, 7.8 %, and 16 % of the coating thickness 58. In an example, the titanium intermediate layer thickness 52 can be within a range of about .0003 µm to about 10 µm such as 0.0004 µm to about 4 µm. For example, the titanium intermediate layer thickness 52 can be within a range of from.01 µm to about 3 µm, such as.2 µm, .6 µm, and 1.7 µm. In an example, the titanium intermediate layer thickness 52 can be about 1 to about 10 times the adhesive layer thickness 56.

The top layer 46 can be formed on at least a portion of the intermediate layer 44. The top layer 46 can increase the surface area of the coating 43, which can increase electrical properties, such as capacitance and admittance and improve the surface finish (e.g., color). The capacitance can increase with an increase in surface area and the capacitance can decrease with dielectric thickness. Increased capacitance improves the signal transduction properties of the film and improves utility for in vivo electrodes.

The control of color can improve the aesthetic appearance of the device. It is of economic utility to manufacture medical devices with consistent appearances, as end users can reject overt deviations in appearance and color from an abundance of caution given the criticality of medical devices to sustain and promote health.

The top layer 46 can include, but is not limited to, porous metals, metal oxides, and metal nitrides. For example, the top layer 46 can include, but is not limited to, porous ruthenium, porous vanadium, porous iridium, porous tantalum, porous niobium, porous tungsten, porous molybdenum, porous palladium, porous platinum, porous titanium, ruthenium oxide, ruthenium nitride, titanium oxide, vanadium oxide, vanadium nitride, iridium nitride, iridium oxide, tantalum nitride, tantalum oxide, niobium nitride, niobium oxide, tungsten nitride, titanium nitride, tungsten oxide, titanium oxide, molybdenum oxide, molybdenum nitride, palladium oxide, palladium nitride, platinum oxide, platinum nitride, and combinations thereof. In an example, the top layer 46 can include a metal-nitride material such as titanium nitride and the top layer 46 can be referred to herein as the "titanium nitride top layer 46."

The top layer 46 can have a small grain size. The term "small grain size" as used herein refers to an average or mean grain size that is less than about 0.125 µm. In an example, the top layer 46 includes a grain size within a range of about 0.005 µm to about 2.0 µm. For example, the average grain size of the top layer 46 can be within a range of about .001 µm to about .5 µm, such as .01 µm, .05 µm and . 1 µm. The average grain size of the top layer 46 can be less than the average grain size of the base layer 42.

As discussed herein, a layer of, for example, titanium nitride having a large grain size can provide increased capacitance but decreased color and abrasion characteristics (e.g., abrasion resistance), as compared to a layer of, for example, titanium nitride having a small grain size. A layer of titanium nitride having a small grain size can provide increased color and abrasion characteristics but a decrease in capacitance, as compared to a layer of titanium nitride having a large grain size. Thus, the coating 43 of the present disclosure incorporates the base layer 42 including large grain sizes and the top layer 46 including small grain sizes. Since the large grain sizes can affect electrical properties but can decrease surface properties (e.g., color and abrasion resistance), the base layer 42 including the large grain sizes is positioned as a middle layer. Since the small grain sizes can affect surface properties such as color and abrasion resistance, the top layer 46 including the small grain size, is positioned as a top layer. Additionally, the small grain sizes can increase the surface area of the coating 43 and thereby further increase the electrical properties of the coating 43.

In an example, the top layer 46 can have a top layer thickness 50 within a range of about .001 % to about 65 % of the coating thickness 58. For example, the top layer thickness 50 can be within a range of about 0.5 % to about 35 %, such as 3 %, 6.5 %, and 12.2 %. In an example, the titanium nitride top layer thickness 50 can be within a range of about .0004 µm to about 8 µm. For example, titanium nitride top layer thickness 50 can be within a range of from .05 µm to about 4.0 µm, such as 0.3 µm, 0.5 µm, 0.7 µm, 1.0 µm, 1.5 µm, and 2.0 µm.

FIG. 3 illustrates generally an example of a method 100 of coating an implantable medical device. In describing the method 100 reference is made to features and elements previously described herein, including numbered references. Numbered elements provided within the description of the method 100 are not intended to be limiting, instead numbered references are provided for convenience and can include any similar features described herein, as well as their equivalents.

The method 100 can include coating an IMD such as a housing 18, as illustrated in FIG. 1. The method 100, at 102, can include depositing a titanium base layer onto a portion of a surface of an IMD. For example, the titanium base layer can be the adhesion layer 40 deposited onto the metallic surface 48 of the IMD 41, as shown in FIG. 2. In an example, depositing the titanium base layer can include depositing substantially pure titanium onto the portion or the surface of the IMD.

The method 100, at 104, can include depositing a titanium nitride base layer onto a portion of the adhesion layer. For example, the method 100 can include depositing the titanium nitride base layer 42 onto the portion of the titanium base layer, as shown in FIG. 2. The method 100, at 106 can include depositing a titanium intermediate layer onto a portion of the titanium nitride base layer. For example, the method 100 can include depositing the titanium intermediate layer 44 onto the portion of the titanium nitride base layer 42, as shown in FIG. 2. In an example, depositing the titanium intermediate layer can include depositing substantially pure titanium onto the portion of the titanium nitride base layer. The method 100, at 108, can include depositing a titanium nitride top layer on at least a portion of the titanium intermediate layer. For example, the method 100 can include depositing the titanium nitride top layer 46 on at least a portion of the titanium intermediate layer 44, as shown in FIG. 2. Depositing can include at least one of sputtering, chemical vapor deposition, and electrochemical processing.

In an example, the coating can include less than four layers. For example, a two or three layer coating can provide advantages to electrical and mechanical performance over a single layer coating or a device that does not include a coating. In an example, the coating can include two layers. For example, the coating can include the adhesion layer and the base layer or the base layer and the top layer, as described herein.

In an example, the coating can include three layers. In an example, the coating can include the base layer, the intermediate layer, and the top layer. In an example, the coating can include the adhesion layer, the base layer, and the intermediate layer. In an example, the coating can include the adhesion layer, the base layer, and the top layer.

In an example, depending on the substrate, sufficient plasma cleaning can provide sufficient adhesion to not include certain layers. For example, in an example where plasma cleaning provides sufficient adhesion, the adhesion layer or the intermediate layer can be removed. However, the two or three layer coatings not including the adhesion layer and/or intermediate layer can result in coatings with reduced abrasion resistance and a general lack of design and process flexibility, as compared to the four layer coating as described herein.

### EXAMPLES

The following examples are given to illustrate, but not limit, the scope of the present disclosure.

### Post-Shock Recovery Time Test Method

Each IMD formed in Examples 1-6 and Comparative Examples 1-2, described below, where fabricated assemblies having a coating are submerged in a saline bath fitted with a counter electrode and reference electrode. A current pulse is delivered through the coated assembly. The post pulse residual potential is measured across the electrodes. The potential magnitude, decay time, and potential derivative are measured at intervals determined by the IMD system functional requirements.

### Electrochemical Impedance Spectroscopy Test Method

Each IMD formed in Example 1 and Comparative Example 1, described below, where fabricated assemblies having a coating are immersed in a saline bath equipped with counter electrodes and reference electrodes. Controlled AC currents are passed between the counter and reference electrodes and the current and voltage data are recorded from the reference electrodes. The frequency is swept across a relevant function range dependent upon the IMD application and the test article geometry.

### Example 1: Forming an IMD having a coating including an adhesive layer, a titanium nitride base layer, a titanium intermediate layer, and a titanium nitride top layer

A pulse generator housing formed of titanium is cleaned with deionized water and placed into a sputter chamber including a solid titanium target and an Argon/Nitrogen gas mixture. An adhesion layer (e.g., titanium base layer) is formed on a surface of the pulse generator can with 5000 watts (W) power and at 15 millitorr (mTorr) pressure. A titanium nitride base layer is formed on a portion of the adhesion layer with 5000 W power, a nitrogen flow rate of 30 standard cubic centimeters per minute (seem), and an argon flow rate of 70 sccm for about 30 minutes. A titanium intermediate layer is formed on a portion of the titanium nitride base layer with 5000 W power, an argon pressure of about 15millitorr for about 5 minutes. A titanium nitride top layer is formed on a portion of the titanium intermediate layer with 5000 W power, a nitrogen flow rate of 140 sccm, and an argon flow rate of 140 sccm for about 10 minutes.

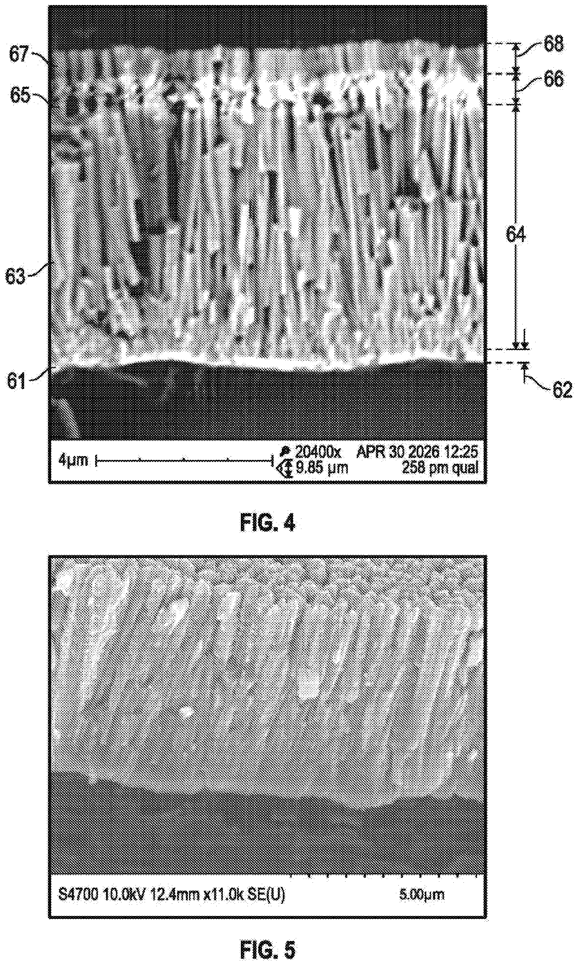

FIG. 4 illustrates a cross-section SEM image of a portion of an IMD. For example, FIG. 4 illustrates the cross-section SEM image of Example 1. As illustrated in FIG. 4, the IMD includes the coating having an adhesive layer 61, a titanium nitrate base layer 63, a titanium intermediate layer 65, and a titanium nitride top layer 67. The coating can have an overall thickness of 7.07 µm, where an adhesive layer thickness 62 is about 0.24 µm, a titanium nitrate intermediate layer thickness 64 is about 5.7 µm, a titanium intermediate layer thickness 66 is about 0.46 µm, and a titanium nitride layer thickness 68 is about 0.67.

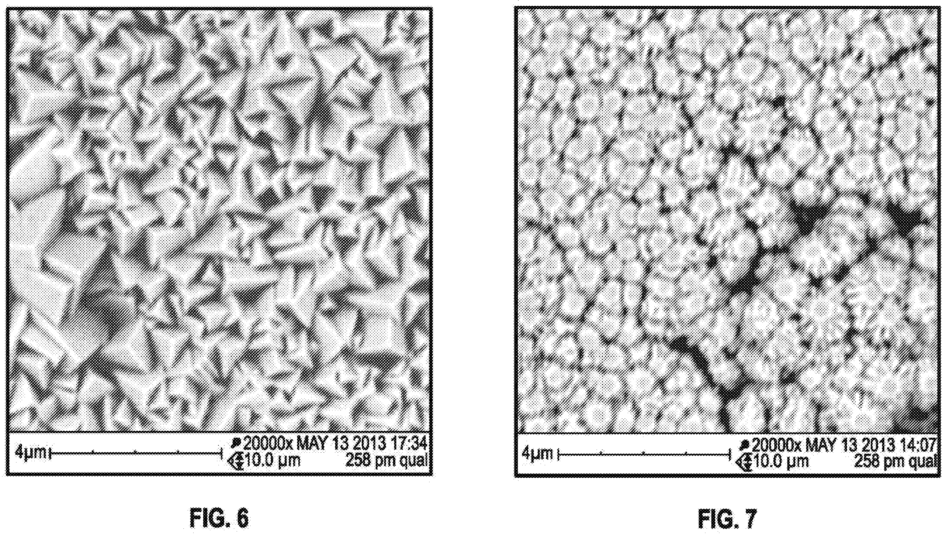

FIG. 5 illustrates a cross-section SEM image of a titanium nitrate base layer and FIG. 6 illustrates a top view SEM image of the titanium nitrate base layer. As illustrated in FIGS. 5 and 6, the titanium base layer includes columnar, pyramidal titanium nitrate grains.

FIG. 7 illustrates a top view SEM image of the IMD including the coating. For example, FIG 7 illustrates a top view of Example 1. As illustrated in FIG. 7, the surface area of coating is increased and the addition of the top layer adds complexity and surface area to the multiple faces of the titanium coated base layer.

After the coating was applied to the housing the housing is ready for inclusion into an IMD assembly processes. For example, the housing including the coating are inserted into an IMD fabrication line where the housing is filled with components, mated with a second case half, and welded to provide a hermetic housing.

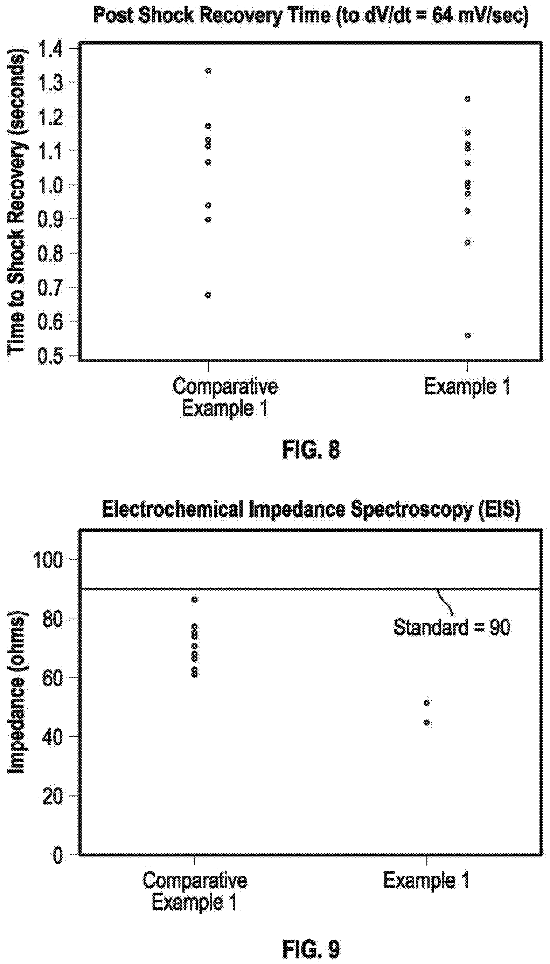

The electrochemical impedance spectroscopy (EIS) of Example 1 was determined for the coating on the housing. The housing including the coating of Example 1 was assembled, as discussed herein, and the post-shock recovery for Example 1 was determined. The results for the post-shock recovery and the EIS are shown in FIGS. 8 and 9, respectively.

### Examples 2-6: Forming various IMDs having a coating including an adhesive layer, a titanium nitride base layer, a titanium intermediate layer, and a titanium nitride top layer

The thickness of the various layers contained it the coating were examined. For example, IMDs including an adhesion layer, titanium nitrate base layer, a titanium intermediate layer, and a titanium top layer were formed according to Example 1, but having the following thickness as shown in Table I.

**Table I**

| | **Example 2** | **Example 3** | **Example 4** | **Example 5** | **Example 6** |
|---|---|---|---|---|---|
| **Adhesion Layer Thickness (µm)** | 0.2 | 0.2 | 0.2 | 0.2 | 0.2 |
| **Titanium Nitride Base Layer Thickness (µm)** | 6.3 | 5.1 | 6.6 | 6.9 | 6.2 |
| **Titanium Intermediate layer Thickness (µm)** | 0.6 | 0.6 | 0.4 | 0.2 | 0.8 |
| **Titanium Nitride Top Layer Thickness (µm)** | 0.5 | 1.9 | 0.5 | 0.5 | 0.5 |

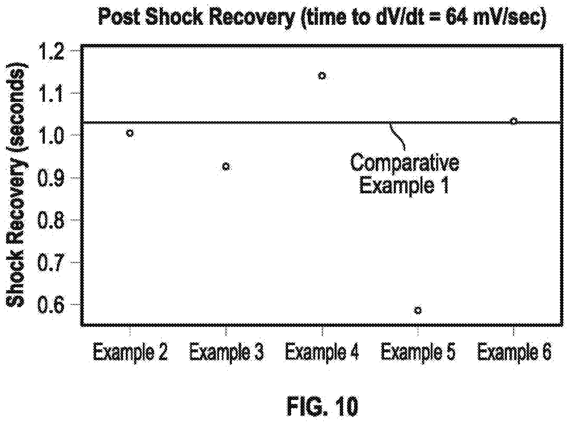

The post-shock recovery times of Examples 2-6 were determined and the results are shown in FIG. 10.

### Comparative Example 1: Forming IMDs including a coating

A pulse generator housing formed of titanium is cleaned with deionized water and placed into a sputter chamber including a solid titanium target and an Argon/Nitrogen gas mixture. A single homogenous titanium nitride layer having a thickness of 7.5 µm is formed onto a surface of the pulse generator can with 7000 W, 25 millitorr pressure, a nitrogen flow rate of 140sccm, and an argon flow rate of 140 sccm for about 45 minutes.

The post-shock recovery and the electrochemical impedance spectroscopy of Comparative Example 1 was determined and the results are shown in FIGS. 8 and 9, respectively.

### Comparative Example 2: Forming IMDs not including a coating

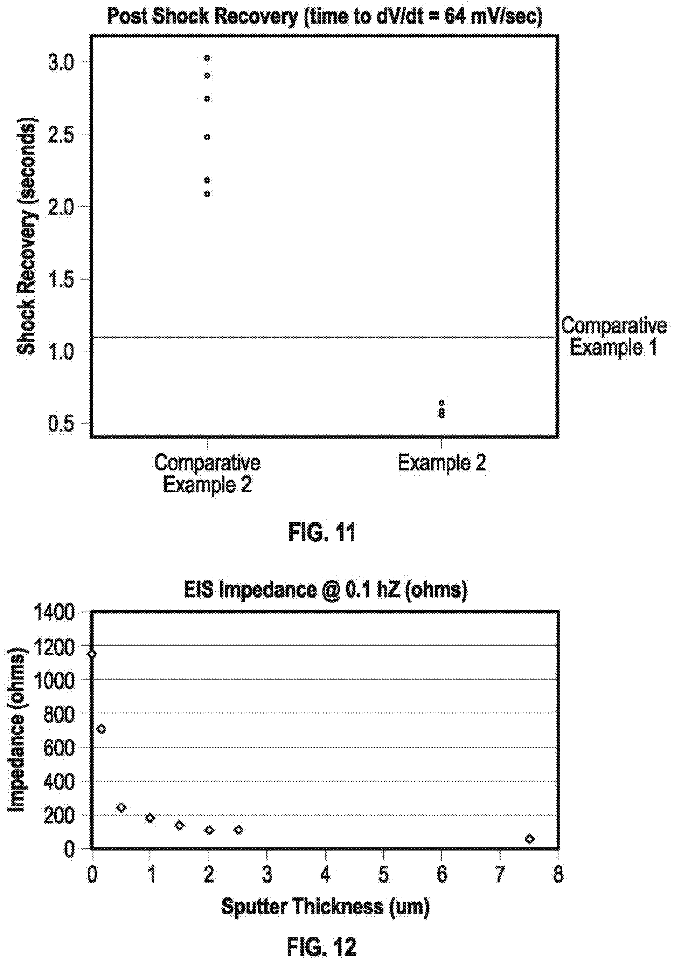

A pulse generator housing formed of titanium that does not include a coating is provided. The post-shock recovery of Comparative Example 2 was determined and the results are shown in FIG. 11, respectively.

### Thickness of Coating Effects on EIS

FIG. 12 illustrates a graph representing the effect of a coating thickness on EIS. As shown in FIG. 12, as the thickness of the coating (e.g., sputter thickness) increases, the impedance decreases.

### Results

FIG. 8 illustrates the post-shock recovery time of Example 1 and Comparative Example 1. As illustrated in FIG. 8, Example 1 can provide substantially the same and/or reduced post-shock recovery times. For example, Example 1 can provide a post-shock recovery time of less than 2 seconds. In an example, the post-shock recovery can be less than 1.4 seconds. In some examples, the post-shock recovery can be less than 1.0 seconds. The post-shock recovery time data demonstrate the enhanced capacitance of the coated surfaces. Reducing the post-shock recovery times are useful as they enable IMDs to rapidly resume effective electronic sensing of cardiac, neurological, and other bioelectric signals following a stimulus event such as a defibrillation shock or pacing pulse.

FIG. 9 illustrates the electrochemical impedance spectroscopy (EIS) of Example 1 and Comparative Example 1. Currently, the industry standard maximum for EIS is 90 ohms. As seen in FIG. 9, Example 1 and Comparative Example 1 are below the industry standard. However, Example 1 has reduced EIS impedance as compared to Comparative Example 1. The measured EIS impedance is an easily performed evaluation with a result that is inversely related to the capacitance of the coated IMD. Therefore, rapid evaluations of coatings can be assessed with lower impedances correlating with the desirable characteristic of high capacitance.

FIG. 10 illustrates the effects on the post-shock recovery times for Examples 2-6 relative the base line performance for Comparative Example 1. As illustrated in FIG. 10, when the thicknesses of various layers of the coating are changed, the post shock recovery time can change. For example, the shock recovery time is insensitive to the adhesive layer thicknesses. The adhesion layer acts as an extension of the can and promotes adhesion between the can and the subsequent coating layer (e.g., the base layer). The thickness of the base layer has a positive correlation to short recovery times. For example, Example 5 includes the thickest titanium nitride base layer and has the lowest post shock recovery time. The thickness of top layer has a positive correlation with short recovery times. That is, as the thickness of the top layer increases, the post shock recovery decreases.

FIG. 11 illustrates the post-shock recovery time of Example 2 and Comparative Example 2, as well as the base line performance for Comparative Example 1. As illustrated in FIG. 11, Example 2 can provide a post shock recovery time less than the Comparative Example 1. For example, Example 2 can provide a post-shock recovery time of less than 1 second. Comparative Example 2 can provide a post shock recovery time of greater than 2 seconds. As discussed herein, reducing the post-shock recovery times are useful as they enable IMDs to rapidly resume effective electronic sensing of cardiac, neurological, and other bioelectric signals following a stimulus event such as a defibrillation shock or pacing pulse.

## Claims

1. (Original) An implantable medical device (41), comprising:
a metallic housing (60); and
a coating (43) formed on at least a portion of the metallic housing (60), the coating (43), including:
an adhesion layer (40) formed on at least a portion of the metallic housing;
a titanium nitride base layer (42) formed on at least a portion of the adhesion layer (40);
a titanium intermediate layer (44) formed on at least a portion of the titanium nitride base layer (42); and
a titanium nitride top layer (46) formed on at least a portion of the titanium intermediate layer (44).

2. (Original) The implantable medical device (41) of claim 1, wherein the adhesion layer (40) and the intermediate layer (44) are substantially pure titanium.

3. (Original) The implantable medical device (41) of claims 1 or 2, wherein a grain size of the titanium nitride base layer (42) is greater than a grain size of the titanium nitride top layer (46).

4. (Original) The implantable medical device (41) of any one of claims 1-3, wherein a titanium nitride base layer thickness (54) is greater than an adhesion layer thickness (56), a titanium intermediate layer thickness (52), and a titanium nitride top layer thickness (50).

5. (Original) The implantable medical device (41) of any one of claims 1-4, wherein a titanium nitride top layer thickness (50) is greater than an adhesion layer thickness (56) and a titanium intermediate layer (52) thickness.

6. (Original) The implantable medical device (41) of any one of claims 1-5, wherein:
a coating thickness is within a range of about 0.25 micrometers to about 34.0 micrometers;
the adhesion layer (40) has a thickness (56) within a range of about 0.0004 micrometers to about 2 micrometers;
the titanium nitride base layer (42) has a thickness (54) within a range of about 0.002 micrometers to about 20 micrometers;
the titanium intermediate layer (44) has a thickness (52) within a range of about 0.0004 micrometers to about 4.0 micrometers; and
the titanium nitride top layer (46) has a thickness (50) within a range of about 0.0004 micrometers to about 8 micrometers.

7. (Currently Amended) An implantable medical device (41), comprising an electrode having an electrode surface, the electrode surface being a metal surface, said device further including a coating (43) formed on at least a portion of the electrode surface, the coating (43) including:
an adhesion layer (40) formed on at least a portion of the electrode surface;
a base layer (42) formed on at least a portion of the adhesion layer (40);
an intermediate layer (44) including titanium formed on at least a portion of the base layer (42); and
a top layer (46) formed on at least a portion of the intermediate layer (44),
wherein the top layer includes porous ruthenium, porous vanadium, porous iridium, porous tantalum, porous niobium, porous tungsten, porous molybdenum, porous palladium, porous platinum, porous titanium, ruthenium oxide, ruthenium nitride, vanadium nitride, iridium nitride, iridium oxide, tantalum nitride, niobium nitride, niobium oxide, tungsten nitride, titanium nitride, tungsten oxide, molybdenum oxide, molybdenum nitride, palladium oxide, palladium nitride, platinum oxide or platinum nitride.

8. (Original) The implantable medical device (41) of claim 7, wherein the adhesion layer (40) includes at least one of substantially pure titanium, ruthenium, vanadium, iridium, tantalum, niobium, tungsten, molybdenum, palladium, platinum, and chromium.

9. (Original) The implantable medical device (41) of either of claims 7 or 8, wherein the base layer (42) includes at least one of porous metals, metal oxides, and metal nitrides.

10. (Original) The implantable medical device (41) of any one of claims 7-9, wherein the intermediate layer (44) includes at least one of substantially pure titanium, ruthenium, vanadium, iridium, tantalum, niobium, tungsten, molybdenum, palladium, platinum, and chromium.

11. (Original) The implantable medical device (41) of any one of claims 7-10, wherein the adhesion layer (40) and the intermediate layer (44) include substantially pure titanium and the base layer (42) and the top layer (46) include titanium nitride.

12. (Original) A method (100) of coating an implantable medical device (41), the method comprising:
depositing an adhesion layer (40) including titanium on at least a portion of a surface of an implantable medical device (41) (102);
depositing a titanium nitride base layer (42) on at least a portion of the adhesion layer (40) (104);
depositing an intermediate layer (44) including titanium on at least a portion of the titanium nitride base layer (42) (106); and
depositing a titanium nitride top layer (46) formed on at least a portion of the intermediate layer (44) (108).

13. (Original) The method of claim 12, wherein depositing includes at least one of sputtering, chemical vapor deposition, and electrochemical processing.

14. (Original) The method of either of claims 12 or 13, wherein depositing the titanium base layer (42) includes depositing substantially pure titanium onto the portion of the surface of the implantable medical device (41), and wherein depositing the titanium cohesive layer includes depositing pure titanium onto the portion of the titanium nitride base layer (42).

## Patentansprüche

1. Implantierbare medizinische Vorrichtung (41), welche aufweist:
ein metallisches Gehäuse (60); und
eine Beschichtung (43), die auf zumindest einem Bereich des metallischen Gehäuses (60) gebildet ist, welche Beschichtung (43) enthält:
eine Adhäsionsschicht (40), die auf zumindest einem Bereich des metallischen Gehäuses gebildet ist;
eine Titannitrid-Basisschicht (42), die auf zumindest einem Bereich der Adhäsionsschicht (40) gebildet ist;
eine Titanzwischenschicht (44), die auf zumindest einem Bereich der Titannitrid-Basisschicht (42) gebildet ist; und
eine Titannitrid-Oberschicht (46), die auf zumindest einem Bereich der Titanzwischenschicht (44) gebildet ist.

2. Implantierbare medizinische Vorrichtung (41) nach Anspruch 1, bei der die Adhäsionsschicht (40) und die Zwischenschicht (44) im Wesentlichen reines Titan sind.

3. Implantierbare medizinische Vorrichtung (41) nach Anspruch 1 oder 2, bei der eine Korngröße der Titannitrid-Basisschicht (42) größer als eine Korngröße der Titannitrid-Oberschicht (46) ist.

4. Implantierbare medizinische Vorrichtung (41) nach einem der Ansprüche 1 - 3, bei der eine Titannitrid-Basisschichtdicke (54) größer als eine Adhäsionsschichtdicke (56), eine Titanzwischenschichtdicke (52) und eine Titannitrid-Oberschichtdicke (50) ist.

5. Implantierbare medizinische Vorrichtung (41) nach einem der Ansprüche 1 - 4, bei der eine Titannitrid-Oberschichtdicke (50) größer als eine Adhäsionsschichtdicke (56) und eine Titanzwischenschichtdicke (52) ist.

6. Implantierbare medizinische Vorrichtung (41) nach einem der Ansprüche 1 - 5, bei der:
eine Beschichtungsdicke innerhalb eines Bereichs von etwa 0,25 Mikrometer bis etwa 34,0 Mikrometer liegt;
die Adhäsionsschicht (40) eine Dicke (56) innerhalb eines Bereichs von etwa 0,0004 Mikrometer bis etwa 2 Mikrometer hat;
die Titannitrid-Basisschicht (42) eine Dicke (54) in einem Bereich von etwa 0,002 Mikrometer bis etwa 20 Mikrometer hat;
die Titanzwischenschicht (44) eine Dicke (52) in einem Bereich von etwa 0,0004 Mikrometer bis etwa 4,0 Mikrometer hat; und
die Titannitrid-Oberschicht (46) eine Dicke (50) in einem Bereich von etwa 0,0004 Mikrometer bis etwa 8 Mikrometer hat.

7. Implantierbare medizinische Vorrichtung (41), aufweisend eine Elektrode mit einer Elektrodenoberfläche, welche Elektrodenoberfläche eine Metalloberfläche ist, wobei die Vorrichtung weiterhin eine Beschichtung (43) enthält, die auf zumindest einem Bereich der Elektrodenoberfläche gebildet ist, wobei die Beschichtung (43) enthält:
eine Adhäsionsschicht (40), die auf zumindest einem Bereich der Elektrodenoberfläche gebildet ist;
eine Basisschicht (42), die auf zumindest einem Bereich der Adhäsionsschicht (40) gebildet ist;
eine Titan enthaltende Zwischenschicht (44), die auf zumindest einem Bereich der Basisschicht (42) gebildet ist; und
eine Oberschicht (46), die auf zumindest einem Bereich der Zwischenschicht (44) gebildet ist,
wobei die Oberschicht poröses Ruthenium, poröses Vanadium, poröses Iridium, poröses Tantal, poröses Niobium, poröses Wolfram, poröses Molybdän, poröses Palladium, poröses Platin, poröses Titan, Rutheniumoxid, Rutheniumnitrid, Vanadiumnitrid, Iridiumnitrid, Iridiumoxid, Tantalnitrid, Niobiumnitrid, Niobiumoxid, Wolframnitrid, Titannitrid, Wolframoxid, Molybdänoxid, Molybdännitrid, Palladiumoxid, Palladiumnitrid, Platinoxid oder Platinnitrid enthält.

8. Implantierbare medizinische Vorrichtung (41) nach Anspruch 7, bei der die Adhäsionsschicht (40) zumindest eines von im Wesentlichen reinem Titan, Ruthenium, Vanadium, Iridium, Tantal, Niobium, Wolfram, Molybdän, Palladium, Platin und und Chrom enthält.

9. Implantierbare medizinische Vorrichtung (41) nach einem der Ansprüche 7 oder 8, bei der die Basisschicht (42) zumindest eines von porösen Metallen, Metalloxiden und Metallnitriden enthält.

10. Implantierbare medizinische Vorrichtung (41) nach einem der Ansprüche 7 - 9, bei der die Zwischenschicht (44) zumindest eines von im Wesentlichen reines Titan, Ruthenium, Vanadium, Iridium, Tantal, Niobium, Wolfram, Molybdän, Palladium, Platin und Chrom enthält.

11. Implantierbare medizinische Vorrichtung (41) nach einem der Ansprüche 7 - 10, bei der die Adhäsionsschicht (40) und die Zwischenschicht (44) im Wesentlichen reines Titan enthalten und die Basisschicht (42) und die Oberschicht (46) Titannitrid enthalten.

12. Verfahren (100) zum Beschichten einer implantierbaren medizinischen Vorrichtung (41), welches Verfahren aufweist:
Aufbringen einer Adhäsionsschicht(40) enthaltend Titan auf zumindest einen Bereich einer Oberfläche einer implantierbaren medizinischen Vorrichtung (41) (102);
Aufbringen einer Titannitrid-Basisschicht (42) auf zumindest einen Bereich der Adhäsionsschicht (40) (104);
Aufbringen einer Zwischenschicht (44) enthaltend Titan auf zumindest einen Bereich der Titannitrid-Basisschicht (42) (106); und
Aufbringen einer Titannitrid-Oberschicht (46), die auf zumindest einem Bereich der Zwischenschicht (44) gebildet ist (108).

13. Verfahren nach Anspruch 12, bei dem das Aufbringen zumindest eines von Sputtern, chemischer Dampfabscheidung und elektrochemischer Verarbeitung enthält.

14. Verfahren nach einem der Ansprüche 12 oder 13,
bei dem das Aufbringen der Titanbasisschicht (42) das Aufbringen von im Wesentlichen reinem Titan auf den Bereich der Oberfläche der implantierbaren medizinischen Vorrichtung (41) enthält, und bei dem das Aufbringen der Titankohäsionsschicht das Aufbringen von reinem Titan auf den Bereich der Titannitrid-Basisschicht (42) enthält.

## Revendications

1. Dispositif médical implantable (41), comprenant :
un boîtier métallique (60) ; et
un revêtement (43) qui est formé sur au moins une partie du logement métallique (60), le revêtement (43) incluant :
une couche d'adhérence (40) qui est formée sur au moins une partie du boîtier métallique ;
une couche de base en nitrure de titane (42) qui est formée sur au moins une partie de la couche d'adhérence (40) ;
une couche intermédiaire en titane (44) qui est formée sur au moins une partie de la couche de base en nitrure de titane (42) ; et
une couche sommitale en nitrure de titane (46) qui est formée sur au moins une partie de la couche intermédiaire en titane (44).

2. Dispositif médical implantable (41) selon la revendication 1, dans lequel la couche d'adhérence (40) et la couche intermédiaire (44) sont sensiblement en titane pur.

3. Dispositif médical implantable (41) selon la revendication 1 ou 2, dans lequel une taille de grain de la couche de base en nitrure de titane (42) est plus importante qu'une taille de grain de la couche sommitale en nitrure de titane (46).

4. Dispositif médical implantable (41) selon l'une quelconque des revendications 1 à 3, dans lequel une épaisseur de couche de base en nitrure de titane (54) est plus importante qu'une épaisseur de couche d'adhérence (56), qu'une épaisseur de couche intermédiaire en titane (52) et qu'une épaisseur de couche sommitale en nitrure de titane (50).

5. Dispositif médical implantable (41) selon l'une quelconque des revendications 1 à 4, dans lequel une épaisseur de couche sommitale en nitrure de titane (50) est plus importante qu'une épaisseur de couche d'adhérence (56) et qu'une épaisseur de couche intermédiaire en titane (52).

6. Dispositif médical implantable (41) selon l'une quelconque des revendications 1 à 5, dans lequel :
une épaisseur de revêtement s'inscrit à l'intérieur d'une plage qui va d'environ 0,25 micromètre à environ 34,0 micromètres;
la couche d'adhérence (40) présente une épaisseur (56) qui s'inscrit à l'intérieur d'une plage qui va d'environ 0,0004 micromètre à environ 2 micromètres ;
la couche de base en nitrure de titane (42) présente une épaisseur (54) qui s'inscrit à l'intérieur d'une plage qui va d'environ 0,002 micromètre à environ 20 micromètres ;
la couche intermédiaire en titane (44) présente une épaisseur (52) qui s'inscrit à l'intérieur d'une plage qui va d'environ 0,0004 micromètre à environ 4,0 micromètres ; et
la couche sommitale en nitrure de titane (46) présente une épaisseur (50) qui s'inscrit à l'intérieur d'une plage qui va d'environ 0,0004 micromètre à environ 8 micromètres.

7. Dispositif médical implantable (41), comprenant une électrode qui comporte une surface d'électrode, la surface d'électrode étant une surface en métal, ledit dispositif incluant en outre un revêtement (43) qui est formé sur au moins une partie de la surface d'électrode, le revêtement (43) incluant :
une couche d'adhérence (40) qui est formée sur au moins une partie de la surface d'électrode ;
une couche de base (42) qui est formée sur au moins une partie de la couche d'adhérence (40) ;
une couche intermédiaire (44) qui inclut du titane et qui est formée sur au moins une partie de la couche de base (42) ; et
une couche sommitale (46) qui est formée sur au moins une partie de la couche intermédiaire (44) ; dans lequel :
la couche sommitale inclut du ruthénium poreux, du vanadium poreux, de l'iridium poreux, du tantale poreux, du niobium poreux, du tungstène poreux, du molybdène poreux, du palladium poreux, du platine poreux, du titane poreux, de l'oxyde de ruthénium, du nitrure de ruthénium, du nitrure de vanadium, du nitrure d'iridium, de l'oxyde d'iridium, du nitrure de tantale, du nitrure de niobium, de l'oxyde de niobium, du nitrure de tungstène, du nitrure de titane, de l'oxyde de tungstène, de l'oxyde de molybdène, du nitrure de molybdène, de l'oxyde de palladium, du nitrure de palladium, de l'oxyde de platine ou du nitrure de platine.

8. Dispositif médical implantable (41) selon la revendication 7, dans lequel la couche d'adhérence (40) inclut au moins un élément chimique sensiblement pur pris parmi le titane, le ruthénium, le vanadium, l'iridium, le tantale, le niobium, le tungstène, le molybdène, le palladium, le platine et le chrome.

9. Dispositif médical implantable (41) selon soit la revendication 7, soit la revendication 8, dans lequel la couche de base (42) inclut au moins soit des métaux poreux, soit des oxydes de métaux et soit des nitrures de métaux.

10. Dispositif médical implantable (41) selon l'une quelconque des revendications 7 à 9, dans lequel la couche intermédiaire (44) inclut au moins un élément chimique sensiblement pur pris parmi le titane, le ruthénium, le vanadium, l'iridium, le tantale, le niobium, le tungstène, le molybdène, le palladium, le platine et le chrome.

11. Dispositif médical implantable (41) selon l'une quelconque des revendications 7 à 10, dans lequel la couche d'adhérence (40) et la couche intermédiaire (44) incluent du titane sensiblement pur et la couche de base (42) et la couche sommitale (46) incluent du nitrure de titane.

12. Procédé (100) de revêtement d'un dispositif médical implantable (41), le procédé comprenant :
le dépôt d'une couche d'adhérence (40) qui inclut du titane sur au moins une partie d'une surface d'un dispositif médical implantable (41) (102) ;
le dépôt d'une couche de base en nitrure de titane (42) sur au moins une partie de la couche d'adhérence (40) (104) ;
le dépôt d'une couche intermédiaire (44) qui inclut du titane sur au moins une partie de la couche de base en nitrure de titane (42) (106) ; et
le dépôt d'une couche sommitale en nitrure de titane (46) qui est formée sur au moins une partie de la couche intermédiaire (44) (108).

13. Procédé selon la revendication 12, dans lequel le dépôt inclut au moins un dépôt pris parmi une pulvérisation, un dépôt chimique en phase vapeur et un traitement électrochimique.

14. Procédé selon soit la revendication 12, soit la revendication 13, dans lequel le dépôt de la couche de base en titane (42) inclut le dépôt de titane sensiblement pur sur la partie de la surface du dispositif médical implantable (41), et dans lequel le dépôt de la couche cohésive en titane inclut le dépôt de titane pur sur la partie de la couche de base en nitrure de titane (42).
